Europäisches Patentamt

European Patent Office

Office européen des brevets

(11) Publication number: **0 284 361**
A1

# (12) EUROPEAN PATENT APPLICATION

(21) Application number: 88302529.8

(22) Date of filing: 23.03.88

(51) Int. Cl.⁴: **A 61 F 5/01**
**A 43 B 3/16**

(30) Priority: 24.03.87 GB 8706933

(43) Date of publication of application:
28.09.88 Bulletin 88/39

(84) Designated Contracting States:
BE DE FR LU NL SE

(71) Applicant: **Johnson & Johnson Products Inc.**
**One Johnson & Johnson Plaza**
**New Brunswick New Jersey 08903 (US)**

(72) Inventor: **Borroff, Michael John**
**Manor Farm Barn Calton**
**Skipton North Yorkshire BD23 4AD (GB)**

(74) Representative: **Fisher, Adrian John et al**
**CARPMAELS & RANSFORD 43 Bloomsbury Square**
**London WC1A 2RA (GB)**

(54) An overshoe for an orthopaedic cast.

(57) An overshoe is provided for an orthopaedic cast such as a plaster cast. The overshoe comprises a sole, an upper piece connected to the sole and a toe cap. The upper piece has first and second parts adapted to receive a plaster cast in the overshoe and has fastening means, such as a "Velcro" (Trade Mark) fastening, allowing those parts to be fastened together to hold the cast firmly within the overshoe. The toe cap is made of such soft material (eg. polyurethane) that it may easily be cut manually, by use of a knife or scissors, to open the toe cap for access to the toes of the wearer.

FIG. 3.

**Description**

AN OVERSHOE FOR AN ORTHOPAEDIC CAST

This invention relates to an overshoe for an orthopaedic cast, for example an overshoe having water resistant uppers.

According to the invention, an overshoe for an orthopaedic cast is provided, comprising a sole, an upper piece connected to the sole, having first and second parts (uppers) adapted to receive the foot portion of an orthopaedic foot cast and having fastening means allowing them to be fastened together to hold the cast firmly within the overshoe, and a toe cap, extending, in use, over the wearer's toes, made of such soft material that it may easily be cut manually, by use of a knife or scissors, to open the toe cap for access to the toes of the wearer.

It is preferred that the sole is connected to the upper piece and/or the toe cap around the entire circumference of the sole in a watertight manner. The upper piece may comprise left and right parts which may overlap along the front of the overshoe and the underside of one of said left and right parts may be removably adhereable to the upper side of the other part, e.g. by means of a "Velcro" (Trade Mark) fastening.

Any semi-rigid, rubbery plastics material capable of being moulded into the appropriate shape, may be used for the toe cap, e.g. polyurethane, polyvinyl chloride shoe moulding materials, vulcanised rubber (as used in rubber Wellington boots) or synthetic thermoplastic rubber based formulations for moulding.

Preferably, the toe cap is not directly attached to the uppers (though of course it is connected to them via the sole), so that a single cut in a horizontal plane just above the level of the upper surface of the sole suffices to remove the toe cap. In this case, it is especially desirable for the uppers to overlap the rearmost edge of the toe cap or for them to overlap a tongue attached to the toe cap to provide improved weather resistance. The latter solution is particularly preferred, with the tongue being unattached to the uppers so that it can be removed with the toe cap if such is desired.

It is preferred that the toe cap is no more difficult to cut than in the case where it is made of polyurethane, having a Shore 'A' hardness of about 60 and a thickness of about 4mm. More preferably, the toe cap at least is made of polyurethane having a Shore 'A' hardness of about 40 to about 60 and a thickness of about 2mm to about 4mm.

In a particularly preferred embodiment, the sole, toe cap and upper piece are moulded of a resilient rubber or plastics material, as a unitary piece. If a tongue is provided, it is preferably stitched to the toe cap.

A preferred embodiment of the invention will now be described, by way of example, with reference to the accompanying drawings, in which:

Fig. 1 is a front perspective view of an overshoe according to the invention, with the left and right parts of the upper piece in an open position;

Fig. 2 is a front perspective view of the overshoe of Figure 1, with the said parts in a closed position;

Fig. 3 is a front perspective view of the overshoe of Figure 2, with the toe cap and tongue removed; and

Fig. 4 is a cross-section, in elevation, of the toe region of the overshoe of Figure 2.

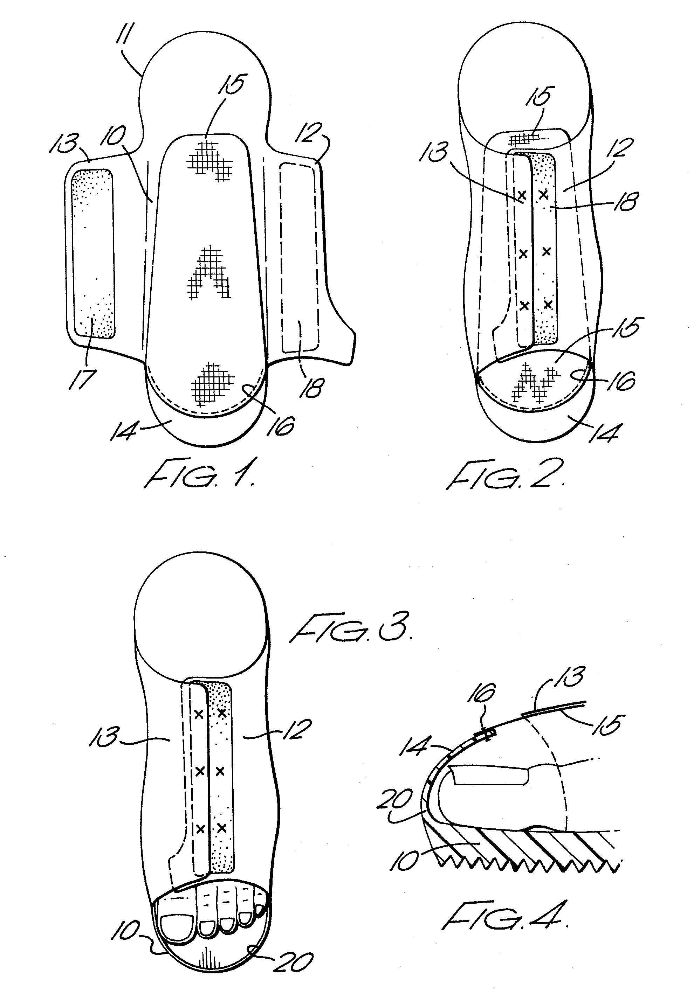

Referring to Fig. 1, the overshoe shown comprises a sole 10, an upper piece 11, having first and second parts in the form of left and right uppers 12 and 13 respectively, a toe cap 14, and a tongue 15. The sole 10, uppers 12 and 13 and toe cap 14 are all formed as a one-piece moulding, of resilient rubber or plastics material, such as polyurethane. The tongue 15, made of canvas, cloth, resilient rubber or plastics material, is a separate piece, but is stitched to the toe cap 14 along the line 16. A "Velcro" fastener 17 is fixed to the inside surface of upper 13 and to the outer surface of upper 12 (see Figure 2).

A patient's foot, encased in a plaster cast, or similar bandage, is placed in the overshoe, so that the toe cap 14 and tongue 15 extend over the toes of the patient and over the top of the foot and its cast. Upper 12 is wrapped over tongue 15 and upper 13 is wrapped over upper 12. The two areas of "Velcro" fastener 17 and 18 stick together and firmly hold the overshoe over the cast.

In this configuration, the overshoe provides excellent water repellant properties to protect the cast. For many patients, this will be the way in which the overshoe is worn throughout the life of the cast. A patient may, however, have inflamed or lacerated toes, or may require surgery to the toes, while the cast is in place. The toes may be infected, and may require swabbing with antiseptic, renewal of dressings, or similar treatment. Other situations where a toe piece may be undesirable include any clinical situations where it is desired to exercise the toes or to relieve pressure on the toes while still allowing the patient no abulate or where Kirchner wires have been used in an internal fixation for a metatarsal osteotomy or a toe fusion. In such cases, it may be unacceptable to use an overshoe having a toe cap.

The present invention can still be used for such patients by the simple step of cutting off the toe cap 14. This can be done with a sharp knife or a pair of scissors, simply by piercing the toe cap 14 and cutting it along the line 20, where it meets the sole 10. From Figure 4, it can be seen that in the region of the toe cap, the overshoe progresses from being very thick in the sole 10, where a tread is moulded into the overshoe, to being quite thin, possibly in the range 1 to 10mm thick, though preferably in the range 2 to 4mm thick, at the line 20 where the toe cap begins. Because the toe cap and sole are made of resilient rubber or plastics material such as polyurethane, and because the toe cap is thin, the process of cutting the toe cap is very simple. The toe cap 14 and tongue 15 are then removed. The resulting overshoe is shown in Figure 3. Here it can be seen that the sole 10 continues to extend underneath the toes of the patient. The sole therefore continues to serve to protect the toes, keeping them dry and providing support and grip.

The invention can be modified in various ways, for example the upper piece 11 can open at different places, to provide access for insertion of the cast into the overshoe, and the tongue, when present, may be rivetted or glued to the toe cap, or may be integrally moulded therewith.

It will of course, be understood that the above description has been given by way of example only, and that modifications of detail can be made within the scope of the invention.

## Claims

1. An overshoe for an orthopaedic cast, comprising a sole, an upper piece connected to the sole, having first and second parts (uppers) adapted to receive the foot portion of an orthopaedic foot cast in the overshoe and having fastening means allowing them to be fastened together to hold the cast firmly within the overshoe, and a toe cap, extending in use, over the wearer's toes, made of such soft material that it may easily be cut manually, by use of a knife or scissors, to open the toe cap for access to the toes of the wearer.

2. An overshoe according to claim 1 wherein the sole is connected to the upper piece and/or the toe cap around the entire circumference of the sole in a watertight manner.

3. An overshoe according to either of claims 1 and 2 wherein the upper piece comprises left and right parts which overlap along the front of overshoe.

4. An overshoe according to any one of claims 1 to 3 wherein the toe cap is not directly attached to the uppers, other than being connected thereto via the sole, so that a single cut in a horizontal plane just above the level of the upper surface of the sole suffices to remove the toe cap.

5. An overshoe according to 4 wherein the uppers overlap the rearmost edge of the toe cap.

6. An overshoe according to claim 4 having a tongue attached to the toe cap, wherein the tongue is overlapped by the uppers.

7. An overshoe according to any one of the preceding claims, wherein the sole, toe cap and upper piece are molded of a resilient rubber or plastics material as a unitary piece.

8. An overshoe according to claim 7 having a tongue according to claim 6 stiched, rivetted or glued to the toe cap.

9. An overshoe according to any one of the preceding claims wherein the underside of one of the first and second parts of the upper piece is removably adherable to the upper-side of the other part.

0284361

FIG. 1.

FIG. 2.

FIG. 3.

FIG. 4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-4 314 412 (B. ANDERSON)<br>--- | 1 | A 61 F 5/01<br>A 43 B 3/16 |
| A | US-A-4 178 703 (S. POLS)<br>----- | 1 | |
| | | | **TECHNICAL FIELDS SEARCHED (Int. Cl.4)**<br><br>A 61 F<br>A 43 B |

The present search report has been drawn up for all claims

| Place of search | Date of completion of the search | Examiner |
|---|---|---|
| THE HAGUE | 03-05-1988 | DECLERCK J.T. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P0401)